(19) Europäisches Patentamt
European Patent Office
Office européen des brevets

(11) Veröffentlichungsnummer: **0 175 652**
**A2**

(12) # EUROPÄISCHE PATENTANMELDUNG

(21) Anmeldenummer: **85810422.7**

(22) Anmeldetag: **16.09.85**

(51) Int. Cl.[4]: **C 07 D 239/42**, C 07 D 401/12, C 07 D 405/12, C 07 D 409/12, A 01 N 43/54

(30) Priorität: **20.09.84 CH 4495/84**
**05.06.85 CH 2382/85**

(43) Veröffentlichungstag der Anmeldung: **26.03.86**
**Patentblatt 86/13**

(84) Benannte Vertragsstaaten: **BE CH DE FR GB IT LI NL**

(71) Anmelder: **CIBA-GEIGY AG, Klybeckstrasse 141, CH-4002 Basel (CH)**

(72) Erfinder: **Zondler, Helmut, Dr., Oberwilerstrasse 49, CH-4103 Bottmingen (CH)**
Erfinder: **Tobler, Hans, Dr., Baselmattweg 157, CH-4123 Allschwil (CH)**
Erfinder: **Müller, Urs, Dr., Drosselstrasse 6, CH-4142 Münchenstein (CH)**

(54) **5-Acylaminopyrimidine.**

(57) Es werden neue 5-Acylaminopyrimidine der allgemeinen Formel I

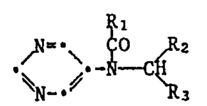

(I)

worin
$R_1$ für Niederalkyl, Halogenniederalkyl, Niederalkoxy, Niederalkoxyniederalkyl, Thioalkyl, Halogen; Phenyl oder Phenoxy, gegebenenfalls jeweils substituiert durch Halogen, Halogenniederalkyl, Carbalkoxyniederalkyl, Halogenniederalkoxy, Niederalkoxy, Phenoxy, Halogenphenoxy, Halogenniederalkylthio, Cyano, Nitro, Niederalkenyl, Niederalkinyl, Cycloalkyl, gegebenenfalls substituiert steht,
$R_2$ Niederalkyl, gegebenenfalls substituiert durch Halogen, Cyano oder Niederalkoxy; Phenyl, Phenylniederalkyl, gegebenenfalls substituiert Pyridyl, Thienyl, Furyl, wobei der Kern gegebenenfalls substituiert ist, Pyranyl, Dihydropyranyl, $C_{3-7}$-Cycloalkyl, $C_{3-8}$-Alkenyl und
$R_3$ Wasserstoff oder $C_{1-8}$-Alkyl bedeutet und $R_2$ und $R_3$ mit dem sie verbindenden Kohlenstoffatom einen Cycloalkylrest bilden können, beschrieben, welche als Pflanzenregulatoren wirksam sind. Es werden ferner Mittel, welche als Wirkstoff mindestens eine dieser Substanzen enthalten, die Herstellung dieser Mittel und ihre Verwendung sowie die entsprechenden neuen 5-Aminopyrimidine und die denen zugrundeliegenden neuen Azomethine als Zwischenprodukte beschrieben.

EP 0 175 652 A2

CIBA-GEIGY AG 5-15079/1+2/+

Basel (Schweiz)

5-Acylaminopyrimidine

Vorliegende Erfindung betrifft neue 5-Acylaminopyrimidine als Pflanzenregulatoren, Mittel, welche als Wirkstoff mindestens eine dieser Verbindungen enthalten, die Herstellung dieser Mittel und ihre Verwendung.

Die neuen 5-Acylaminopyrimidine entsprechen der allgemeinen Formel I

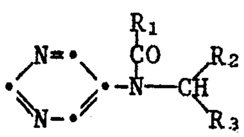

(I)

worin

$R_1$ für $C_{1-8}$-Alkyl, Halogen-$C_{1-6}$-alkyl, $C_{1-6}$-Alkoxy, $C_{1-4}$-Alkoxy-$C_{1-8}$-alkyl, Thio-$C_{1-4}$-alkyl, Halogen; Phenyl oder Phenoxy, gegebenenfalls jeweils substituiert durch Halogen, $C_{1-4}$-Alkyl, Halogen-$C_{1-4}$-alkyl, Carboxy-$C_{1-4}$-alkyl, Halogen-$C_{1-4}$-alkoxy, $C_{1-4}$-Alkoxy, Halogen-$C_{1-4}$-alkylthio, Cyano, Nitro; $C_{2-4}$-Alkenyl, $C_{2-4}$-Alkinyl, $C_{3-6}$-Cykloalkyl, gegebenenfalls substituiert durch Halogen, und $C_{1-4}$-Alkyl, steht,

$R_2$ $C_{1-8}$-Alkyl, gegebenenfalls substituiert durch Halogen, Cyano, $C_{1-4}$-Alkoxy; Phenyl, Phenyl-$C_{1-8}$-alkyl, worin der Phenylkern jeweils gegebenenfalls wie unter $R_1$ definiert, substituiert ist; Pyridyl, Thienyl, Furyl, wobei der Kern wie der Phenylrest unter $R_1$, definiert, gegebenenfalls substituiert ist, Pyranyl, Dihydropyranyl, $C_{3-7}$-Cycloalkyl, $C_{3-7}$-Alkenyl, und

$R_3$ Wasserstoff oder $C_{1-8}$-Alkyl bedeutet und $R_2$ und $R_3$ mit dem sie verbindenden Kohlenstoffatom einen $C_{5-7}$-Cycloalkylrest bilden können.

Unter dem Begriff Alkyl selbst oder als Bestandteil eines anderen Substituenten, wie Alkoxy, Thioalkyl, Alkoxy-alkyl, Halogenalkoxy, Carboxyalkyl, Halogenalkyl, Halogenalkylthio, sind je nach Zahl der angegebenen Kohlenstoffatome beispielsweise folgende Gruppen zu verstehen: Methyl, Aethyl, Propyl, Butyl, Pentyl, Hexyl, Heptyl, Octyl, sowie ihre Isomeren, wie z.B. Isopropyl, Isobutyl, sec.-Butyl, tert.-Butyl, Isopentyl, Isoheptyl usw.. Halogenalkyl steht für einen einfach bis perhalogenierten Alkylsubstituenten, wie z.B. $CHCl_2$, $CHF_2$, $CH_2Cl$, $CCl_3$, $CH_2F$, $CH_2CH_2Cl$, $CHBr_2$ usw. Unter Halogen soll hier und im folgenden Fluor, Chlor, Brom oder Jod, vorzugsweise Fluor, Chlor oder Brom, verstanden werden. Alkenyl bedeutet z.B. Propenyl-(1), Allyl, Butenyl-(1), Butenyl-(2), Butenyl-(3), oder Isoheptenyl, Alkinyl steht z.B. für Aethinyl, Propinyl-(1) oder Propargyl. Cycloalkyl bedeutet je nach Zahl der Kohlenstoffatome z.B. Cyclopropyl, Cyclobutyl, Cyclopentyl, Cyclohexyl oder Cycloheptyl.

Unter dem Begriff Pyridyl sind die Reste 3- und 4-Pyridyl, unter dem Begriff Thienyl die Reste 2- und 3-Thienyl und unter dem Begriff Furyl, der 2-Furylrest, unter dem Begriff Pyranyl die 2H-2-Pyranyl-, 2H-3-Pyranyl, 2H-4-Pyranylreste und unter dem Begriff Dihydropyranyl die 3-(2H-5,6-Dihydropyranyl-) und 3-(2H-3,4-Dihydropyranyl)-Reste zu verstehen.

Acylamino-1,4-Diazine sind als Pflanzenregulatoren bekannt (USP 4,441,912).

Die erfindungsgemässen 5-Acylamino-pyrimidine (d.h. 5-Acylamino-1,3-diazine) sind neu. Diese Verbindungen der Formel I sind bei Raumtemperatur stabile Oele, Harze oder überwiegend Feststoffe, die sich durch sehr wertvolle wuchsregulierende Eigenschaften auszeichnen. Sie lassen sich auf dem Agrarsektor oder verwandten Gebieten präventiv und kurativ zur Regulierung des Pflanzenwuchses einsetzen, wobei die erfindundungsgemässen Wirkstoffe der Formel I sich durch eine sehr gute Verträglichkeit bei Kulturpflanzen auszeichnen.

Aufgrund ihrer ausgeprägten wuchsregulierenden Wirkung sind diejenigen Verbindungen bevorzugt, in denen $R_1$ $C_{2-4}$-Alkyl ist, $R_2$ Phenyl, gegebenenfalls durch Chlor substituiert ist und $R_3$ Wasserstoff ist.

Besonders bevorzugt sind diejenigen Verbindungen, in denen $R_1$ Aethyl, Propyl, Isopropyl oder tert-Butyl ist, $R_2$ 4-Chlorphenyl oder 2,4-Dichlorphenyl ist.

Folgende Einzelverbindungen sind besonders bevorzugt:
N-(4-Chlorbenzyl)-N-(pyrimidin-5-yl)-propionsäureamid,
N-(2,4-Dichlorbenzyl)-N-(pyrimidin-5-yl)-isobuttersäureamid,
N-(4-Chlorbenzyl)-N-(pyrimidin-5-yl)-trimethylacetamid,
N-(2,4-Dichlorbenzyl)-N-(pyrimidin-5-yl)-trimethylacetamid.
N-(4-Brombenzyl)-N-(pyrimidin-5-yl)-trimethylacetamid.

Die 5-Acylaminopyrimidine können durch Umsetzung eines 5-Aminopyrimidins der Formel II, worin $R_2$ und $R_3$ die unter Formel I gegebene Bedeutung haben, mit einem Acylierungsmittel der Formel IV hergestellt werden:

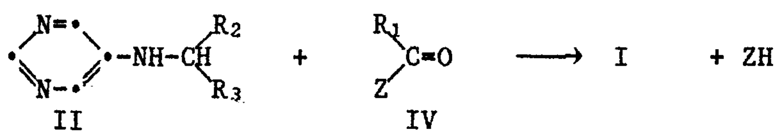

II IV

Als Acylierungsmittel der Formel IV, worin $R_1$ die unter Formel I gegebene Bedeutung hat eignen sich beispielsweise Säurehalogenide (Z = Halogen) oder Säureanhydride (Z = $R_1COO$-), worin $R_1$ die unter Formel I gegebene Bedeutung hat. Die Acylierungsmittel der Formel IV sind allgemein bekannt oder lassen sich nach bekannten Methoden herstellen. Bei der Acylierung mit einem Säurehalogenid kann ein inertes Lösungsmittel und ein Säureakzeptor eingesetzt werden. Als Säureakzeptoren eignen sich tertiäre Amine, welche vorteilhafterweise gegebenenfalls auch als Lösungsmittel dienen können. Bei der Acylierung mit einem Säureanhydrid kann ebenfalls ein inertes Lösungsmittel mit Säureakzeptor eingesetzt werden. Nach einer

vorteilhaften Ausführungsform wird hier die als Nebenprodukt entstehende Säure als Lösungsmittel eingesetzt und gegebenenfalls die katalytische Wirkung von Mineralsäuren, insbesondere von Schwefelsäure genutzt.

Die Herstellung der 5-Aminopyrimidine der Formel II erfolgt aus den Azomethinen (Schiff'schen Basen) der Formel III, worin $R_2$ und $R_3$ die unter Formel I gegebene Bedeutung haben, durch Reduktion, beispielsweise mit Hilfe von Natriumborhydrid oder durch katalytische Hydrierung, in einem geeigneten Lösungsmittel, wie zum Beispiel Tetrahydrofuran oder Methanol:

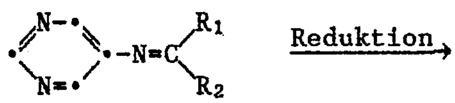


II

III

Die Herstellung der Azomethine der Formel III erfolgt durch Kondensation des 5-Aminopyrimidins der Formel V mit einer Oxoverbindung der Formel VI, worin $R_2$ und $R_3$ die unter Formel I gegebene Bedeutung haben, in einem geeigneten Lösungsmittel, wie zum Beispiel Methanol.

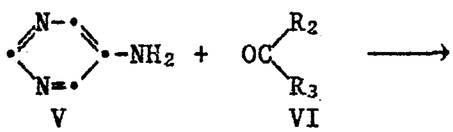

III + $H_2O$

V VI

Die 5-Aminopyrimidine der Formel II und die Azomethine der Formel III sind neu und bilden ebenfalls Gegenstand der vorliegenden Erfindung.

Das 5-Aminoprimidin der Formel V ist aus der Literatur bekannt (vgl. Whittacker, J. Chem. Soc. 1951 p. 1568).

Es wurde nun überraschend gefunden, dass die neuen Wirkstoffe der Formel I bzw. Mittel, die diese Wirkstoffe enthalten, sich vor allem dadurch auszeichnen, dass sie gezielt in den Metabolismus der

Pflanzen eingreifen. Dieser gezielte Eingriff in die physiologischen Vorgänge der Pflanzenentwicklung macht die Wirkstoffe der Formel I für verschiedene Zwecke verwendbar, insbesondere für solche, die mit der Ertragssteigerung bei Nutzpflanzen, der Ernteerleichterung und der Arbeitseinsparung bei Massnahmen an Pflanzenkulturen im Zusammenhang stehen.

Für die Wirkungsweise von Pflanzenwachstumsregulatoren gilt nach der bisherigen Erfahrung, dass ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung ist unter anderem bei Gräsern von wirtschaftlichem Interesse, denn dadurch kann z.B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Ueberlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, so dass z.B. mehr oder grössere Früchte zur Ausbildung kommen.

Ertragssteigerungen können in manchen Fällen auch durch einen Eingriff in den pflanzlichen Stoffwechsel erreicht werden, ohne dass sich Aenderungen des vegetativen Wachstums bemerkbar machen. Wachstumsregulatoren können ferner eine Veränderung der Zusammensetzung der Pflanzen bewirken, so dass eine bessere Qualität der Ernteprodukte herbeigeführt wird. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluss von Wachstumsregulatoren kann es zur Ausbildung parthenokarper Früchte kommen. Ferner kann das Geschlecht der Blüten beeinflusst werden.

Mit Wachstumsregulatoren lässt sich auch die Produktion oder der Abfluss von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z.B. Interesse bei der Stecklingsvermehrung von Pflanzen. Es ist jedoch auch möglich, das Wachstum der Seitentriebe zu hemmen, z.B. um bei Tabakpflanzen nach der Dekapitierung die Ausbildung von Seitentrieben zu verhindern und damit das Blattwachstum zu fördern.

Unter dem Einfluss von Wachstumsregulatoren kann der Blattbestand von Pflanzen so gesteuert werden, dass ein Entblättern der Pflanzen zu einem gewünschten Zeitpunkt erreicht wird. Eine derartige Entlaubung ist von Interesse, um eine mechanische Beerntung z.B. bei Wein oder Baumwolle, zu erleichtern oder um die Transpiration zu einem Zeitpunkt herabzusetzen, an dem die Pflanzen verpflanzt werden soll. Durch Einsatz von Wachstumsregulatoren lässt sich der vorzeitige Fruchtfall verhindern. Es ist jedoch auch möglich, den Fruchtfall, - zum Beispiel bei Obst -, im Sinne einer chemischen Ausdünnung bis zu einem bestimmten Ausmass zu fördern. Wachstumsregulatoren können auch dazu dienen, um bei Kulturpflanzen zum Zeitpunkt der Ernte die zum Ablösen der Früchte erforderliche Kraft zu vermindern, so dass eine mechanische Beerntung der Pflanzen ermöglicht, beziehungsweise eine manuelle Beerntung erleichtert wird.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden Voraussetzungen dafür geschaffen, dass z.B. bei Tabak, Tomaten oder Kaffee, eine vollständige mechanische oder manuelle Beerntung in nur einem Arbeitsgang vorgenommen werden kann.

Durch die Anwendung von Wachstumsregulatoren kann auch die Samen- oder Knospenruhe der Pflanzen, also die endogene Jahresrhythmik, beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, dass der Austrieb von Knospen oder die Keimung von Samen verzögert wird, z.B. um in frostgefährdeten Gebieten eine Schädigung durch Spätfröste zu vermeiden. Andererseits gelingt es, das Wurzelwachstum zu und/oder die Ausbildung von Sprösslingen zu stimulieren, so dass das Wachstum auf eine kürzere Zeitdauer beschränkt werden kann.

Wachstumsregulatoren können auch eine Halophilie bei den Kulturpflanzen erzeugen. Damit werden die Voraussetzungen dafür geschaffen, dass eine Kultivierung von Pflanzen auf salzhaltigen Böden durchgeführt werden kann.

Mit Wachstumsregulatoren kann auch eine Frost- und Trockenresistenz bei Pflanzen induziert werden.

Unter dem Einfluss von Wachstumsregulatoren kann das Altern (die Seneszenz) von Pflanzen oder Pflanzenteilen gehemmt respektive verzögert werden. Eine solche Wirkung kann von hohem wirtschaftlichem Interesse sein, dadurch, dass bei behandelten Pflanzenteilen oder ganzen Pflanzen wie Obst, Beeren, Gemüse, Salat oder Zierpflanzen deren Lagerfähigkeit nach der Ernte verbessert oder verlängert werden kann. Ebenso kann durch Beahndlung von Kulturpflanzen über eine Verlängerung der Phase photosynthetischer Aktivität eine beachtliche Ertragssteigerung erzielt werden.

Ein weiteres wichtiges Anwendungsgebiet für Wuchshemmer ist deren Einsatz zur Hemmung eines übermässigen Wachstums bei tropischen Bodenbeckungspflanzen, den sogenannten Cover crops. In tropischen und subtropischen Monokulturen, wie z.B. in Palmplantagen, Bauwoll-,

Maisfeldern usw. werden neben den eigentlichen Kulturpflanzen oftmals Bodenbedeckungspflanzen, insbesondere Leguminosenarten angepflanzt, die zur Erhaltung oder Steigerung der Bodenqualität (Verhinderung der Austrocknung, Versorgung mit Stickstoff) und zur Verhinderung von Erosion (Abtragung durch Wind und Wasser) dienen. Durch Applikation der erfindungsgemässen Wirkstoffe kann nunmehr das Wachstum dieser Cover crops kontrolliert und somit die Wuchshöhe dieser Bodenbedeckungspflanzen auf einem niedrigen Niveau gehalten werden, so dass ein gesundes Gedeihen der Kulturpflanzen und die Aufrechterhalung einer günstigen Bodenbeschaffenheit gewährleistet ist.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer): oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewaschungen, seien es Zierpflanzen (Compositen),

Grasflächen, Böschungen oder allgemeine niedrige Bodenbedeckungen (cover crops), die einer Erosion oder Austrocknung des Bodens entgegenwirken oder Bodenbedeckungen wie sie in Baum- und Staudenkulturen (Obstplantagen, Hopfenkulturen, Maisfeldern, Weingärten usw.) erwünscht sind.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelment-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlilchen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen richten sich dabei nach der Art der Wachstumsbeeinflussung. Die Wirkstoffe der Forme I können aber auch über den Erdboden durch das Wurzelwerk in die Pflanzen gelangen (systemilsche Wikrung), indem man den Standort der Pflanzen mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstande verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

sie mit einer festen Zubereitung beschichtet. Darüberhinaus sind in besonderen Fällen weitere Applikationsarten möglich, so z.B. die gezielte Beahndlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemiltteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS), je ha; bevorzugt 100 g bis 2 kg AS/ha, insbesondere bei 200 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidiertes Pflanzenöl wie epoxydiertes Kokosnussöl oder Soja, Oel; oder Wasser.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen, Alkylarylsulfonate sind z.B. die Na-, Ca- oder Triäthanolaminsalze der Deodecabenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979.
Sisley and Wood, "Encyclopedia of Surface Active Agents", Chemical Publishing Co., Inc. New York, 1964.

Die pestiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 %, eines Tensides.

Insbesondere setzen sich bevorzugte Formulierungen folgendermassen zusammen: (% = Gewichtsprozent)

Lösungen

| | |
|---|---|
| Aktiver Wirkstoff: | 5 bis 95 %, vorzugsweise 10 bis 80 % |
| Lösungsmittel: | 95 bis 5 %, vorzugsweise 90 bis 0 % |
| oberflächenaktives Mittel: | 1 bis 30 %, vorzugsweise 2 bis 20 % |

Emulgierbare Konzentrate

| | |
|---|---|
| Aktiver Wirkstoff | 10 bis 50 %, bevorzugt 10 bis 40 % |
| oberflächenaktives Mittel: | 5 bis 30 %, vorzugsweise 10 bis 20 % |
| flüssiges Trägermittel: | 20 bis 95 %, vorzugsweise 40 bis 80 % |

Stäube

| | |
|---|---|
| Aktiver Wirkstoff: | 0,5 bis 10 %, vorzugsweise 2 bis 8 % |
| festes Trägermaterial | 99,5 bis 90 %, vorzugsweise 98 bis 92 % |

Suspensions-Konzentrate

| | |
|---|---|
| Aktiver Wirkstoff | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 % |

Benetzbare Pulver

| | |
|---|---|
| Aktiver Wirkstoff | 5 bis 90 %, vorzugsweise 10 bis 80 % |
| und | insbesondere 20 bis 60 % |
| oberflächenaktives Mittel: | 0,5 bis 20 %, vorzugsweise 1 bis 15 % |
| festes Trägermaterial: | 5 bis 90 %, vorzugsweise 30 bis 70 % |

Granulate

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe zu beschränken. Temperaturen sind in Celsiusgraden angegeben.

Herstellungsbeispiele

Beispiel 1a): Herstellung von N-(4-Chlorbenzyl)-N-(pyrimidin-5-yl)-trimethylacetamid. (Verbindung Nr. 1.001)

3,08 g (14 Mmol) N-(4-Chorbenzyl)-N-(pyrimidin5-yl)-amin werden in 20 ml Pyridin und 0,2 g p-Dimethylaminopyridin gelöst und die Lösung wird unter Rühren auf 100° erhitzt. Hierzu wird während 30 Min 3,02 g (25 Mmol) Trimethylacetylchlorid portionsweise zugegeben. Das Reaktionsgemisch wird auf Raumtemperatur abgekühlt und mit 50 ml Aethylacetat verdünnt und mit verdünnter Schwefelsäure stark angesäuert. Das Rühren wird nach 5 Min abgestellt und die Schichten werden getrennt. Nach der Phasentrennung wird das Lösungsmittel von der leichteren Schicht im Vakuum bei 80° verdampft und der resultierende Rückstand von 5,3 g aus einem Gemisch von 3 ml Toluol und 8 ml Cyclohexan umkristallisiert. Das resultierende Reinprodukt schmilzt bei 122-123°, Ausbeute 37,2 %. Weitere 43 % d.Th. N-(4-Chlorbenzyl)-N-(pyrimidin-5-yl)-trimethylacetamid sind nach Aufarbeitung der Mutterlauge (Säulenchromatographie über Kieselgel, Laufmittel 3:2=Hexan:Essigester) erhältlich.

Beispiel 1b): Herstellung des N-(4-Chlorbenzyl)-5-aminopyrimidins. (Verbindung Nr. 2.001).

8,78 g (40 Mmol) N-(4-Chlorbenzyliden)-5-aminopyrimidin, gelöst in 70 ml Methanol wird unter Rühren bei Raumtemperatur während 30 Minuten portionsweise mit 1,89 g (50 Mmol) Natriumborhydrid versetzt. Nach weiteren 30 Minuten wird das Reaktionsgemisch mit 200 ml Wasser und 50 ml Aethylacetat verrührt und das Rühren beendet. Die obere Schicht wird anschliessend abgetrennt und mit Natriumsulfat getrocknet. Aus dem Filtrat wird das Lösungsmittel im Vakuum bei 60° verdampft und der Rückstand aus 25 ml Aethanol umkristallisiert. Es resultieren 4,83 g (54,5 % d.Th.) N-(4-Chorbenzyl)-5-aminopyrimidin vom Schmp. 128-129°. Nach Verdampfen der Mutterlauge und erneuter Umkristallisation des Rückstandes aus Aethanol sind noch 1,99 g (22,5 % d.Th.) des Produkts mit dem Schmelzpunkt von 128-129° erhältlich.

Beispiel 1c): Herstellung von N-(4-Chlor-benzyliden)-5-aminopyrimidin (Verbindung Nr. 3.001)

5,71 g (60 Mmol) 5-Aminopyrimidin und 9,84 g (70 Mmol) 4-Chlorbenzyladehyd werden in 75 ml Methanol vermischt und zum Sieden erhitzt. Das aus der Lösung beim Abkühlen ausgeschiedene Kristallisat wiegt 6,22 g (s47,6 % d.Th.) und schmilzt bei 138-39°. Nach Umkristallisieren des Destillationsrückstands der Mutterlauge aus Aethanol können noch zwei weitere Fraktionen des Produkts 2,69 g (20,6 % d.Th., Smp.: 138-39°) und 3,0 g (23,7 % d.Th., Smp.:137-38°) isoliert werden.

Beispiel 2a): Herstellung des N-(2,4-Dichlorbenzyl)-N-(pyrimidin-5-yl)-acetamids. (Verbindung Nr. 1.002)

2,80 g (11 Mmol) N-(2,4-Dichlorbenzyl)-N-(pyrimidin-5-yl)-amin werden in 5 ml Eisessig gelöst und mit 5 ml Essigsäureanhydrid und 3 Tropfen konz. Schwefelsäure versetzt und 1,5 Stunden lang unter Rühren und unter Rückfluss gekocht. Das Reaktionsgemisch wird anschliessend abgekühlt, mit 30 ml Chloroform versetzt, die Essigsäure wird durch Extraktion mit Wasser entfernt und die abgetrennte Chloroform-Lösung mit Natriumsulfat getrocknet. Das Chloroform wird

nach Abfiltrieren des Natriumsulfats im Vakuum bei 80° verdampft und der Rückstand aus einem Gemisch von 5 ml Toluol und 5 ml Cyclohexan umkristallisiert. Es resultieren 2,23 g (68,5 % d.Th.) N-(2,4-Dichlorbenzyl)-N-(pyrimidin-5-yl)-acetamids, Schmp.: 134-136°. Die Reinigung des im Filtrat enthaltenen Produkts mit einer Kieselgelsäule (Laufmittel Essigester) ergibt weitere 0,90 g (27,6 % d.Th.). Reinprodukt.

Beispiel 2b): Herstellung von N-(2,4-Dichlorbenzyl)-5-aminopyrimidin. (Verbindung Nr. 2.002)

11,06 g (44 Mmol) N-(2,4-Dichlorbenzyliden)-5-aminopyrimidin, verrührt mit 60 ml Methanol und 30 ml Dioxan werden portionsweise mit 2,00 g (53 Mmol) Natriumborhydrid versetzt. Die Temperatur steigt dabei von 20 auf 50° und es entsteht eine Lösung, die mit Wasser und Chloroform verrührt wird. Nach Trocknen der abgetrennten Chloroform-Schicht mit $Na_2SO_4$ wird diese Lösung in Vakuum eingedampft und der Rückstand einer Mischung aus 30 ml Toluol und 5 ml Aethanol auskristallisiert. Das isotierte Produkt wiegt 7,13 g (64 % d.Th.) und schmilzt bei 104-105°. Weitere 3,22 g (28,9 % d.Th.) des Produkts sind nach Säulenchromatographische Reinigung des Verdampfungsrückstandes der Mutterlauge in Methylacetat über Kieselgel und nach der vorhin geschilderten Aufarbeitung erhältlich.

Beispiel 2c: Herstellung des N-(2,4-Dichlorbenzyliden)-5-aminopyrimidins. (Verbinung Nr. 3.002).

5,71 g (60 Mmol) 5-Aminopyrimidin, gelöst in 30 ml Methanol, wird bei Raumtemperatur mit 11,55 g (66 Mmol) 2,4-Dichlorbenzaldehyd, gelöst in 30 ml Methanol, verrührt, und die entstandene Lösung wird unter Rühren zum Kochen erhitzt, wobei das resultierende Produkt auskristallisiert. Das Kristallisat wird bei Raumtemperatur abgesaugt und getrocknet. Das bei 189-191° schmelzende N-(2,4-Dichlorbenzyliden)-5-aminopyrimidin wiegt 14,51 g (95,9 % d.Th.).

In analoger Weise werden die in den folgenden Tabellen angeführten Verbindungen hergestellt:

0175652

Tabelle 1

Verbindungen der Formel I

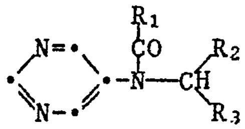

(I)

| Nr. | $R_1$ | $R_2$ | $R_3$ | Physikalische Konstante |
|---|---|---|---|---|
| 1.001 | $(CH_3)_3$-C- | 4-Cl-$C_6H_4$ | H | Smp. 122-123° |
| 1.002 | $CH_3$- | 2-Cl-4-Cl-$C_6H_3$- | H | Smp. 134-136° |
| 1.003 | $(CH_3)_3$-C- | 2-Cl-4-Cl-$C_6H_3$- | H- | Smp. 107-108° |
| 1.004 | $(CH_3)_2$-CH- | 2-Cl-4-Cl-$C_6H_3$- | H- | Smp. 104-105° |
| 1.005 | $C_2H_5$- | 4-Cl-$C_6H_4$- | H- | Smp. 92-94° |
| 1.006 | $(CH_3)_3$-C- | 4-F-$C_6H_4$- | H- | Smp. 104-105° |
| 1.007 | $(CH_3)_3$-C- | 4-$CH_3$-$C_6H_4$- | H- | Smp. 145-146° |
| 1.008 | $(CH_3)_3$-C- | 3-$CH_3$-4-$CH_3$-$C_6H_3$- | H- | |
| 1.009 | $(CH_3)_3$-C- | 2-$CH_3$-4-$CH_3$-$C_6H_3$- | H- | |
| 1.010 | $(CH_3)_3$-C- | 2-Cl-$C_6H_4$- | H- | Smp. 100-102° |
| 1.011 | $(CH_3)_3$-C- | 2-Cl-3-Cl-$C_6H_3$- | H- | |
| 1.012 | $(CH_3)_3$-C- | $C_6H_5$- | H- | Smp. 106-107° |
| 1.013 | $(CH_3)_3$-C- | 3-$CF_3$-$C_6H_4$- | H- | Smp. 106-107° |
| 1.014 | $(CH_3)_3$-C- | 2-F-3-Cl-$C_6H_3$- | H- | |
| 1.015 | $(CH_3)_3$-C- | 2-Br-3-Cl-$C_6H_3$- | H- | |
| 1.016 | $(CH_3)_3$-C- | 2-Cl-4-Br-$C_6H_3$- | H- | |
| 1.017 | $(CH_3)_3$-C- | 4-Br-$C_6H_4$- | H- | Smp. 128-129° |
| 1.018 | 4-Cl-$C_6H_4$- | $C_2H_5$- | H- | |
| 1.019 | 4-Cl-$C_6H_4$- | $CH_3$-$CH_2$-$CH_2$- | H- | |
| 1.020 | 4-Cl-$C_6H_4$- | $(CH_3)_3$C- | H- | |
| 1.021 | 4-Cl-$C_6H_4$- | $(CH_3)_2$CH- | H- | |
| 1.022 | 2-Cl-4-Cl-$C_6H_3$- | $C_2H_5$- | H- | |
| 1.023 | 2-Cl-4-Cl-$C_6H_3$- | $CH_3$-$CH_2$-$CH_2$- | H- | |
| 1.024 | 2-Cl-4-Cl-$C_6H_3$- | $(CH_3)_3$C- | H- | |
| 1.025 | 2-Cl-4-Cl-$C_6H_3$- | $(CH_3)_2$CH- | H- | |

Tabelle 1: (Forsetzung)

| Nr. | $R_1$ | $R_2$ | $R_3$ | Physikalische Konstante |
|---|---|---|---|---|
| 1.026 | $2-Cl-4-Cl-C_6H_3-$ | $4-Cl-C_6H_4-$ | H- | |
| 1.027 | $2-Cl-4-Cl-C_6H_3-$ | $2-Cl-4-Cl-C_6H_3-$ | H- | |
| 1.028 | $(CH_3)_3C-$ | $4-Cl-C_6H_4-CH_2O-$ | H- | |
| 1.029 | $(CH_3)_3C-$ | $4-F-C_6H_4-CH_2O-$ | H- | |
| 1.030 | $(CH_3)_3C-$ | $2-Cl-4-Cl-C_6H_3-CH_2O-$ | H- | |
| 1.031 | $(CH_3)_3C-$ | $C_6H_5-CH_2O-$ | H- | |
| 1.032 | $(CH_3)_2CH-$ | $4-Cl-C_6H_4-CH_2O-$ | H- | |
| 1.033 | $(CH_3)_2CH-$ | $4-F-C_6H_4-CH_2O-$ | H- | |
| 1.034 | $(CH_3)_2CH-$ | $2-Cl-4-Cl-C_6H_3-CH_2O-$ | H- | |
| 1.035 | $(CH_3)_2CH-$ | $(CH_3)_3C-$ | H- | |
| 1.036 | $(CH_3)_2CH-$ | $C_2H_5-$ | H- | |
| 1.037 | $4-Cl-C_6H_4-$ | $4-Cl-C_6H_4-CH_2O-$ | H- | |
| 1.038 | $(CH_3)_3C-$ | $4-Cl-C_6H_4-$ | $CH_3-$ | Smp. 133° |
| 1.039 | $(CH_3)_3C-$ | $(CH_3)C-$ | $CH_3-$ | |
| 1.040 | $(CH_3)_3C-$ | $C_2H_5-$ | $CH_3-$ | |
| 1.041 | $(CH_3)_3C-$ | $4-Cl-C_6H_4-CH_2O-$ | $CH_3-$ | |
| 1.042 | $(CH_3)_3C-$ | $4-F-C_6H_4-CH_2O-$ | $CH_3-$ | |
| 1.043 | $C_6H_4-$ | $(CH_3)_3C-$ | H- | |
| 1.044 | $C_6H_5$ | $4-Cl-C_6H_4-$ | H- | |
| 1.045 | 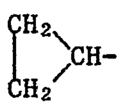 | $4-Cl-C_6H_4-$ | H- | |
| 1.046 | 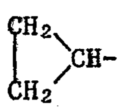 | $2-Cl-4-Cl-C_6H_3-$ | H- | |

Tabelle 1: (Fortsetzung)

| Nr. | $R_1$ | $R_2$ | $R_3$ | Physikalische Konstante |
|---|---|---|---|---|
| 1.047 | 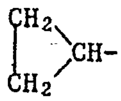 | $C_2H_5$- | H- | |
| 1.048 | 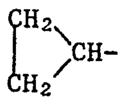 | $(CH_3)_3C$- | H- | |
| 1.049 | 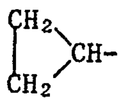 | 4-F-$C_6H_4$- | H- | |
| 1.050 | 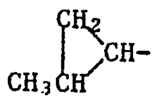 | 4-Cl-$C_6H_4$- | H- | |
| 1.051 | 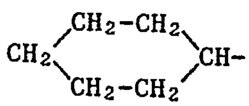 | 4-Cl-$C_6H_4$- | H- | |
| 1.052 | $(CH_3)_3C$- | 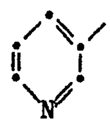 | H- | Smp. 116-117° |
| 1.053 | $(CH_3)_3C$- | 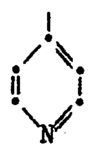 | H- | |
| 1.054 | $(CH_3)_3C$- | 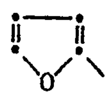 | H- | Smp. 112-114° |
| 1.055 | $(CH_3)_3C$- | 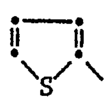 | H- | Smp. 75-77° |
| 1.056 | $(CH_3)_3C$- | 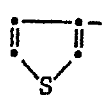 | H- | Smp. 92-93° |
| 1.057 | $CH_3$- | 2-Cl-4-Cl-$C_6H_3$- | H- | Smp. 134-136° |
| 1.058 | $C(CH_3)_3$- | 4-Cl-$C_6H_4$- | H- | Smp. 120-123° |
| 1.059 | $(CH_3)_3C$- | $(-CH_2-)_5$ | | Smp. 133-134° |
| 1.060 | $Cl_2CH$- | $C_6H_5$- | H- | Smp. 127-128° |
| 1.061 | $Cl_2CH$- | 2-Cl-6-Cl-$C_6H_3$- | H- | Smp. 148-149° |
| 1.062 | $Cl_2CH$- | 2-Cl-$C_6H_4$- | | Smp. 164-166° |
| 1.063 | $(CH_3)_3C$- | 4-$(CH_3O)C_6H_4$- | H | Smp. 106° |

Tabelle 1: (Fortsetzung)

| Nr. | $R_1$ | $R_2$ | $R_3$ | Physikalische Konstante |
|---|---|---|---|---|
| 1.064 | $Cl_2CH$ | 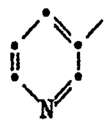 | H | Smp. 125-128° |
| 1.065 | $(CH_3)_3C-$ | 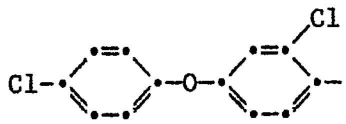 | H- | Smp. 124-125° |
| 1.066 | $Cl_2CH-$ | 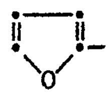 | H- | Smp. 105-107° |
| 1.067 | $(CH_3)_3C-$ | 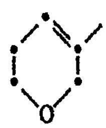 | H- | Smp. 82-84° |
| 1.068 | $(CH_3)_3C-$ | $CH_3-(CH_2)_2-CH=C(C_2H_5-)-$ | H- | Smp. 101-102° |
| 1.069 | $(CH_3)_3C-$ | $(-CH_2-)_6$ | | Smp. 127-129° |
| 1.070 | $(CH_3)_3C-$ | $2-Cl-6-Cl-C_6H_3-$ | H- | Smp. 165-166° |
| 1.071 | $CH_2=C(C_2H_5)-$ | $C_6H_5$ | H- | Smp. 76-77° |
| 1.072 | $CH_2=C(C_2H_5)-$ | $2-Cl-6-Cl-C_6H_3-$ | H- | Smp. 115-116° |
| 1.073 | $4-Cl-C_6H_4-$ | 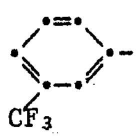 | H- | Smp. 92-94° |
| 1.074 | $(CH_3)_3C-$ | $(CH_3)_3C-$ | H- | Smp. 140-141° |
| 1.075 | $(CH_3)_3C-$ | 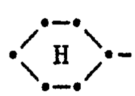 | H- | Smp. 120-122° |
| 1.076 | $(CH_3)_3C-$ | $3-Cl-C_6H_4$ | H- | Smp. 127-128° |
| 1.077 | $CH_3OCH_2-$ | $4-Cl-C_6H_4$ | H | Smp. 77-78° |
| 1.078 | $(CH_3)(C_2H_5)CH-$ | $4-Cl-C_6H_4$ | H | Smp. 101° |

<u>Tabelle 2:</u> Verbindungen der Formel II

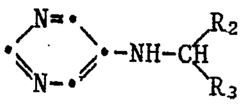

II

| Nr. | $R_2$ | $R_3$ | Physikalische Konstante |
|---|---|---|---|
| 2.001 | H | $4-Cl-C_6H_4-$ | Smp. 128-129° |
| 2.002 | H- | $2-Cl-4-Cl-C_6H_3-$ | Smp. 104-105° |
| 2.003 | H- | $4-F-C_6H_4-$ | Smp. 125-126° |
| 2.004 | H- | $4-CH_3-C_6H_4-$ | Smp. 142-143 |
| 2.005 | H- | $3-CH_3-4-CH_3-C_6H_3-$ | |
| 2.006 | H- | $3-CH_3-4-CH_3-C_6H_3-$ | |
| 2.007 | H- | $2-CH_3-3-CH_3-C_6H_3-$ | |
| 2.008 | H- | $2-Cl-C_6H_4-$ | Smp. 91-93° |
| 2.009 | H- | $2-Cl-3-Cl-C_6H_3-$ | |
| 2.010 | H- | $C_6H_5-$ | Smp. 107-109° |
| 2.011 | H- | $3-CF_3-C_6H_4-$ | Smp. 123-125° |
| 2.012 | H- | $2-F-4-Cl-C_6H_3-$ | |
| 2.013 | H- | $2-Br-4-Cl-C_6H_3-$ | |
| 2.014 | H- | $2-Cl-4-Br-C_6H_3-$ | |
| 2.015 | H- | $2-Br-C_6H_4-$ | |
| 2.016 | H- | $C_2H_5-$ | |
| 2.017 | H- | $CH_3-CH_2-CH_2-$ | |
| 2.018 | H- | $(CH_3)_3C-$ | Smp. 193-195° |
| 2.019 | H- | $(CH_3)_2CH-$ | |
| 2.020 | H- | $4-Cl-C_6H_4-CH_2O-$ | |

Tabelle 2: (Fortsetzung)

| Nr. | $R_2$ | $R_3$ | Physikalische Konstante |
|---|---|---|---|
| 2.021 | H- | $4-F-C_6H_4-CH_2O-$ | |
| 2.022 | H- | $2-Cl-4-Cl-C_6H_3-CH_2O-$ | |
| 2.023 | H- | $C_6H_5-CH_2O-$ | |
| 2.024 | $CH_3-$ | $4-Cl-C_6H_4-$ | Smp. 157-159° |
| 2.025 | $CH_3-$ | $(CH_3)_3C-$ | |
| 2.026 | $CH_3-$ | $C_2H_5-$ | |
| 2.027 | $CH_3-$ | $4-Cl-C_6H_4-CH_2O-$ | |
| 2.028 | $CH_3-$ | $4-F-C_6H_4-$ | |
| 2.029 | $CH_3-$ | 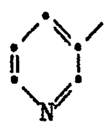 | |
| 2.030 | $CH_3-$ | 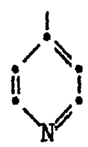 | |
| 2.031 | $CH_3-$ | 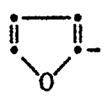 | |
| 2.032 | $CH_3-$ | 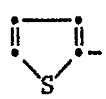 | |
| 2.033 | $CH_3-$ | 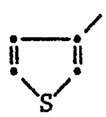 | |
| 2.034 | H | $2,6-di-Cl-C_6H_3$ | Smp. 144-146° |
| 2.035 | $-(CH_2)_5-$ | | Smp. 114-116° |
| 2.036 | H | 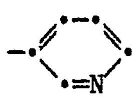 | Smp. 93-95° |
| 2.037 | H | $-CH_2-(CH_2)_2-CH=C(C_2H_5)-$ | Sdp. $10^{-2}$ Torr/112-116° |

<u>Tabelle 2:</u> (Fortsetzung)

| Nr. | $R_2$ | $R_3$ | Physikalische Konstante |
|---|---|---|---|
| 2.038 | H | 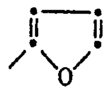 | Smp. 111-113° |
| 2.039 | H | 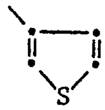 | Smp. 135-137° |
| 2.040 | H | 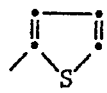 | Smp. 145-146° |
| 2.041 | H | 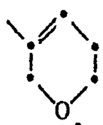 | Smp. 87-89° |
| 2.042 | H | 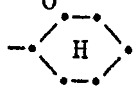 | Smp. 73-74° |
| 2.043 | H | 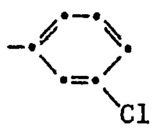 | Smp. 114-116° |
| 2.044 | H | $-(CH_2)_6-$ | Smp. 83-85° |
| 2.045 | H | $4-Cl-C_6H_4-$ | Smp. 128-129° |
| 2.046 | H | $4-Br-C_6H_4-$ | Smp. 139-140° |
| 2.047 | H | $4-CH_3O-C_6H_4-$ | Smp. 166-168° |
| 2.048 | H | 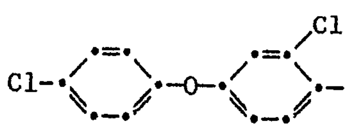 | Smp. 111-113° |

Tabelle 3: Verbindungen der Formel III

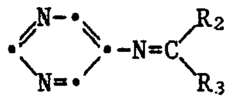

III

| Nr. | $R_2$ | $R_3$ | Physikalische Konstante |
|---|---|---|---|
| 3.001 | H- | 4-Cl-$C_6H_4$- | Smp. 138-139° |
| 3.002 | H- | 2-Cl-4-Cl-$C_6H_3$- | Smp. 189-191° |
| 3.003 | H- | 4-F-$C_6H_4$- | Smp. 102-103° |
| 3.004 | H- | 4-$CH_3$-$C_6H_4$- | Smp.74-76° |
| 3.005 | H- | 3-$CH_3$-4-$CH_3$-$C_6H_3$- | |
| 3.006 | H- | 2-Cl-$C_6H_4$- | Smp. 91-92° |
| 3.007 | H- | 2-Cl-3-Cl-$C_6H_3$- | |
| 3.008 | H- | $C_6H_5$- | Smp. 48-52° |
| 3.009 | H- | 3-$CF_3$-$C_6H_4$- | Sdp. 135°/$10^{-2}$ Torr |
| 3.010 | H- | 2-F-4-Cl-$C_6H_3$- | |
| 3.011 | H- | 2-Br-4-Cl-$C_6H_3$- | |
| 3.012 | H- | 2-Cl-4-Br-$C_6H_3$- | |
| 3.013 | H- | 4-Br-$C_6H_4$- | Smp. 157-158° |
| 3.014 | H- | $CH_3$-$CH_2$-$CH_2$- | |
| 3.015 | H- | $(CH_3)_3C$- | Sdp. 48°/10 Torr |
| 3.016 | H- | $(CH_3)_2CH$- | |
| 3.017 | H- | 4-Cl-$C_6H_4$-$CH_2O$- | |
| 3.018 | H- | 4F-$C_6H_4$-$CH_2O$- | |
| 3.019 | H- | 2-Cl-4-Cl-$C_6H_3$-$CH_2O$- | |
| 3.020 | H- | $C_6H_5$-$CH_2O$- | |
| 3.021 | $CH_3$- | 4-Cl-$C_6H_4$- | Smp. 100-102°C |
| 3.022 | $CH_3$- | $(CH_3)_3C$- | |
| 3.023 | $CH_3$- | $C_2H_5$- | |
| 3.024 | $CH_3$- | 4-Cl-$C_6H_4$-$CH_2O$- | |
| 3.025 | $CH_3$- | 4-F-$C_6H_4$- | |
| 3.026 | $CH_3$- | 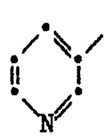 | |

<u>Tabelle 3:</u> Verbindungen der Formel III

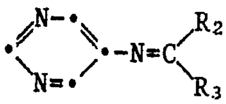

III

| Nr. | $R_2$ | $R_3$ | Physikalische Konstante |
|---|---|---|---|
| 3.027 | $CH_3-$ | 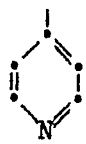 | |
| 3.028 | $CH_3-$ | 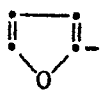 | |
| 3.029 | $CH_3-$ | 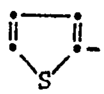 | |
| 3.030 | $CH_3-$ | 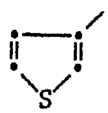 | |
| 3.031 | H | 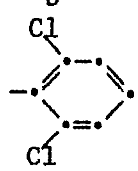 | Smp. 118-120 |
| 3.032 | H | $-(CH_2)_5-$ | Smp. ca. 120°/$10^{-2}$ Torr |
| 3.033 | H | 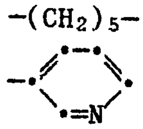 | Smp. 134-135° |
| 3.034 | H | $CH_3-(CH_2)_2-CH=C(C_2H_5)$ | Sdp. 100-103°/$10^{-1}$ Torr |
| 3.035 | H | 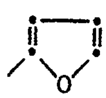 | Smp. 118-120° |
| 3.036 | H | 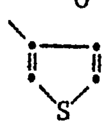 | Smp. 81-83° |
| 3.037 | H | 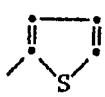 | Smp. 80-82° |
| 3.038 | H | 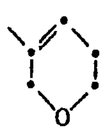 | Smp. 79-81° |
| 3.039 | H | 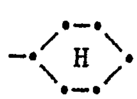 | viskoses Oel |

Tabelle 3: (Fortsetzung)

| Nr. | $R_2$ | $R_3$ | Physikalische Konstante |
|---|---|---|---|
| 3.040 | H | 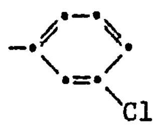 | Smp. 69-70° |
| 3.041 | H | $-(CH_2)_6-$ | Sdp. ca. 130°/$10^{-2}$ Torr |
| 3.042 | H | 2-Cl-4-Cl-$C_6H_3-$ | Smp. 189-191° |
| 3.043 | H | 4-$CH_3O$-$C_6H_4-$ | Smp. 128-131° |
| 3.044 | H | 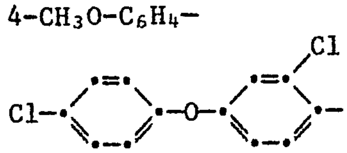 | Smp. 125-127° |

Formulierungsbeispiele

Beispiel 3:

Formulierungsbeispiele für flüssige Wirkstoffe der Formel I

(% = Gewichtsprozent)

a) Emulsionskonzentrate

| | a) | b) | c) |
|---|---|---|---|
| Wirkstoff | 20% | 40% | 50% |
| Ca-Dodecalbenzolsulfonat | 5% | 8% | 5,8% |
| Ricinusöl-polyäthylenglykoläther (36 Mol AeO) | 5% | - | - |
| Tributylphenoyl-polyäthylenglykoläther (30 Mol AeO) | - | 12% | 4,2% |
| Cyclohexanon | - | 15% | 20% |
| Xylolgemisch | 70% | 25% | 20% |

Aus solchen Konzentraten können durch Verdünnen mit Wasser Emulsionen jeder gewünschten Konzentration hergestellt werden.

b) Lösungen

| | a) | b) | c) | d) |
|---|---|---|---|---|
| Wirkstoff | 80% | 10% | 5% | 95% |
| Aethylenglykol-monomethyl-äther | 20% | - | - | - |
| Polyäthylenglykol M G 400 | - | 70% | - | - |
| N-Methyl-2-pyrrolidon | - | 20% | - | - |
| Expoxidiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

c) Granulate

| | a) | b) |
|---|---|---|
| Wirkstoff | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| d) Stäubemittel | a) | b) |
|---|---|---|
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Beispiel 4: Formulierungsbeispiele für Wirkstoffe der Formel I (% = Gewichtsprozent)

| a) Spritzpulver | a) | b) |
|---|---|---|
| Wirkstoff | 20 % | 60 % |
| Na-Ligninsulfonat | 5 % | 5 % |
| Na-Laurylsulfat | 3 % | - |
| Na-Diisobutylnaphthalinsulfonat | - | 6% |
| Octylphenolpolyäthylenglykoläther /7-8 Mol AeO) | - | 2 % |
| Hochdisperse Kieselsäure | 5 % | 27 % |
| Kaolin | 67 % | - |
| Natriumchlorid | - | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | |
|---|---|
| Wirkstoff | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

d) Extruder Granulat

| | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | |
|---|---|
| Wirkstoff | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 5: Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt umgerechnet 0,5 bzw. 2,5 kg Aktivsubstanz pro Hektar. 10 und 21 Tage nach Applikation wird das Wachstum des Getreides beurteilt. Hierbei kann festgestellt werden, dass Getreidepflanzen, die mit Wirkstoffen der Formel I behandelt worden sind, im Vergleich zu unbehandelten Kontrollpflanzen eine starke Wuchsreduktion aufweisen. Als besonders wirksam erweisen sich Verbindungen aus der Tabelle 1. So reduzieren die Verbindungen Nr. 1.003-1.005 die Zuwachsrate auf weniger als 10 %.

Beispiel 6: Wuchshemmung bei Gräsern

In Kunststoffschalen mit Erde-Torf-Sand-Gemisch (6:3:1) werden die Gräser Lölium perenne, Poa pratensis, Festuca ovina und Cynodon dactylon im Gewächshaus angesät und nach Bedarf bewässert. Die aufgelaufenen Gräser werden wöchentlich bis auf ca. 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet 0,5 bzw. 2,5 kg Aktivsubstanz pro Hektar. 10 und 21 Tage nach Applikation wird das Wachstum der Gräser beurteilt, dabei zeigt es sich, dass die erfindungsgemässen Wirkstoffe aus der Tabelle 1 eine merkliche Wuchshemmung bewirken. Besonders deutliche Wuchshemmung wird mit der Verbindung Nr. 1.003 bewirkt, so reduziert diese Verbindung das Weiterwachsen fast vollständig (Zuwachsrate 0 bis 10 %).

Beispiel 7: Ertragssteigerung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifola-Blattstadium. Zu diesem Zeitpunkt werden die Pflanzen mit der wässrigen Brühe eines Wirkstoffes der Formel I bis zur guten Benetzung besprüht. Die Wirkstoffkonzentration beträgt bis zu 500 ppm Aktivsubstanz. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der geernteten Schoten. Als besonders wirksam erweisen sich die Verbindungen aus der Tabelle 1. Insbesondere die Verbindungen Nr. 1.003-1.005 bewirken eine Ertragssteigerung von 5 bis 12 %.

Beispiel 8: Wuchshemmung bei Bodendecker-Pflanzen (Cover-Crops)
In Kunststoffschalen mit Erde-Torf-Sand-Gemisch (1:1:1) werden Testpflanzen der Sorte Centrosema plumieri und Centrosema pubescens aus Stecklingen angezogen. Nach dem Anwurzeln werden die Pflänzchen in 9-cm-Töpfe umgetopft und nach Bedarf gewässert. Die weitere Anzucht der Pflanzen findet im Gewächshaus bei einer Tagestemperatur von 27°C und einer Nachttemperatur von 21°C, bei einer mittleren Lichtdauer von 14 h (6000 Lux) und einer Luftfeuchtigkeit von 70 % statt. Die Testpflanzen werden auf eine Höhe von ca. 15 cm zurückgeschnitten und 7 Tage nach dem Zurückschneiden mit einer Spritzbrühe des Wirkstoffes (umgerechnet 0,3 bzw. 3 kg Aktivsubstanz je Hektar) besprüht. 4 Wochen nach Applikation wird das Wachstum der behandelten Pflanzen im Vergleich zu gestutzten, aber unbehandelten Kontrollpflanzen verglichen. Hierbei kann festgestellt werden, dass Verbindungen aus der Tabelle 1 eine deutliche Wuchshemmung der Bodenbedecker-Pflanzen auslösen. Insbesondere die Verbindungen 1.003-1.005 bewirken eine starke Wuchshemmung und reduzieren die Zuwachsrate auf 0 bis 10 %.

Beispiel 9: Seneszenzhemmung bei Getreidepflanzen
Im Gewächshaus wird Sommerweizen der Sorte "Svenno" in Töpfen mit Komposterde angesät und ohne spezielle klimatische Anforderungen gezogen. Ca. 10 Tage nach dem Auflaufen werden 10 bis 12 cm lange Primärblätter abgeschnitten und einzeln in Reagenzgläser mit 10 ml einer Wirkstoffsuspension (1,25 bis 10 ppm Aktivsubstanz) gegeben. Die Reagenzgläser werden in einem klimatisierten Raum bei 23°C, 70 % relativer Luftfeuchtigkeit aufgestellt und täglich durchschnittlich 14 Stunden (10 000 Lux) bestrahlt. Die Beurteilung der Seneszenzhemmung erfolgt 7 Tage nach Einstellen der Blätter durch Vergleich des Vergilbungsgrades im Verhältnis zu noch frischen, grünen Blättern. Bei diesem Versuch kann beobachtet werden, dass Verbindungen aus der Tabelle 1 eine deutliche Inhibierung der Seneszenz der Testpflanzen hervorrufen. Insbesondere die Verbindungen Nr. 1.003-100.5, hemmen das Vergilben der Blätter im Versuchszeitraum um mehr als 80 %.

Beispiel 10: Wuchshemmung bei Getreide

In Kunststofftöpfen (Ø 12 cm) mit sterilisierter Erde werden Gerste und Weizen im Gewächshaus angesät und Bedarf bewässert. Die Pflanzen werden auf 10 Stück pro Topf ausgedünnt. Die Sprösslinge werden ca. 21 Tage nach der Aussaat mit der wässrigen Spritzbrühe eines Wirkstoffes der Formel I besprüht. Die Wirkstoffmenge beträgt umgerechnet 0,3, 1,0 und 3,0 kg Aktivsubstanz pro Hektar. Vier Wochen nach Applikation wird das Wachstum des Getreides beurteilt. Es ergibt sich folgendes Resultat:

| Behandlung mit Ver-bindung Nr. | Menge in kg AS/ha | Kultur | Neuwuchs nach Behandlung Pflanzen-höhe | Trocken-gewicht | % Trocken-gewicht / % Höhe | Stil-Umfang von 10 Pflan-zen |
|---|---|---|---|---|---|---|
| unbehandelt | | | 100 % | 100 % | 1.0 | 100 % |
| 1.017 | 0.3 | Gerste | 95 % | 100 % | 1.1 | 103 % |
| 1.017 | 1.0 | Gerste | 81 % | 70 % | 0.9 | 106 % |
| 1.017 | 3.0 | Gerste | 51 % | 64 % | 1.3 | 136 % |
| 1.017 | 0.3 | Weizen | 45 % | 80 % | 1.8 | 104 % |
| 1.017 | 1.0 | Weizen | 31 % | 75 % | 2.4 | 112 % |
| 1.017 | 3.0 | Weizen | 14 % | 73 % | 5.2 | 127 % |
| 1.001 | 0.3 | Gerste | 44 % | 99 % | 2.3 | 102 % |
| 1.001 | 1.0 | Gerste | 33 % | 72 % | 2.2 | 106 % |
| 1.001 | 3.0 | Gerste | 19 % | 60 % | 3.2 | 122 % |
| 1.001 | 0.3 | Weizen | 82 % | 79 % | 1.0 | 112 % |
| 1.001 | 1.0 | Weizen | 55 % | 59 % | 1.1 | 115 % |
| 1.001 | 3.0 | Weizen | 29 % | 52 % | 1.8 | 124 % |

Beispiel 11: Wuchshemmung an Sojabohnen

7 Sojapflanzen/Kunststoffbehälter (Variante "Williams") werden in einem Erde-Torf-Sandgemisch im Verhältnis 6:3:1 gezüchtet. Die Pflanzen werden unter Triebhausbedingungen gehalten und nach Bedarf bewässert.

2 Wochen nach ihrer Aussaat werden die Pflanzen mit der wässrigen Brühe eines Wirkstoffs der Formel I zur guten Benetzung besprüht. Die Wirkstoffmenge entspricht 0,1, 0,5 und 1,5 mg Aktivsubstanz pro Hektar. 2 Wochen nach Applikation wird der Neuwuchs der Pflanzen ermittelt und mit dem Neuwuchs der unbehandelten Pflanzen verglichen. Es ergibt sich folgendes Resultat:

| Behandlung mit Verbindung Nr. | Menge in kg AS/ha | Kultur | Neuwuchs nach Behandlung Höhe | Frischgewicht | % Gewicht / % Höhe |
|---|---|---|---|---|---|
| unbehandelt | | | 100 % | 100 % | 1.0 |
| 1.003 | 0.1 | Sojabohnen | 50 % | 89 % | 1.8 |
| 1.003 | 0.5 | Sojabohnen | 27 % | 83 % | 3.1 |
| 1.003 | 1.5 | Sojabohnen | 23 % | 71 % | 3.1 |
| 1.001 | 0.1 | Sojabohnen | 100 % | 100 % | 1.0 |
| 1.001 | 0.5 | Sojabohnen | 86 % | 95 % | 1.1 |
| 1.001 | 1.5 | Sojabohnen | 76 % | 95 % | 1.3 |
| 1.017 | 0.1 | Gerste | 100 % | 100 % | 1.0 |
| 1.017 | 0.5 | Gerste | 81 % | 94 % | 1.2 |
| 1.017 | 1.5 | Gerste | 44 % | 84 % | 1.9 |

Beispiel 12: Wuchshemmung bei Gräsern

Eine hauptsächlich aus Gräsern (Poa pratensis, Dactylis glomerata, Lolium perenne, Festuca rubra, Festuca ovina, Cynosurus cristatus, Agropyron repens, Promus inermis) bestehendes Gemisch und etwas Klee werden in Kunststoffbehältern vorgelegte sterilisierte Erde gesät, im Gewächshaus gehalten und nach Bedarf bewässert. Die aufgelaufenen Gräser werden wöchentlich bis auf ca. 4 cm Höhe zurückgeschnitten und nach 8-9 Wochen nach der Aussaat und einen Tag nach dem letzten Schnitt mit der wässrigen Spritzbrühe der Wirkstoffe der Formel I besprüht. Die Wirkstoffkonzentration beträgt 0,3, 1,0 und 3,0 kg Wirksubstanz pro Hhektar. Wochen nach der Applikation der Wirkstoffe ergibt die Auswertung folgendes Resultat:

| Behandlung mit<br>Verbindung Nr. | Menge in kg AS/ha | Kultur | Neuwuchs nach Behandlung (Pflanzenhöhe) |
|---|---|---|---|
| Unbehandelt | | | 100 % |
| 1.017 | 0.3 | Grasgemisch | 91 % |
| 1.017 | 1.0 | Grasgemisch | 79 % |
| 1.017 | 3.0 | Grasgemisch | 68 % |
| 1.001 | 0.3 | Grasgemisch | 94 % |
| 1.001 | 1.0 | Grasgemisch | 81 % |
| 1.001 | 3.0 | Grasgemisch | 79 % |

Beispiel 13: Wuchshemmung bei Bodendecker-Pflanzen (Cover-Crops)

In Kunststoffbehältern (Ø 12 cm) mit Erde-Torf-Gemisch (1:3) werden gut angewurzelte Stecklinge der Testpflanzen der Sorte Centrosema plumieri und Centrosema pubescens gepflanzt. Die Testpflanzen werden auf eine Höhe von ca. 15 cm zurückgeschnitten und 7 Tage nach dem Zurückschneiden mit einer Spritzbrühe des Wirkstoffes (umgerechnet 0,3, 1 und 3 kg Aktivsubstanz je Hektar) besprüht. 4 Wochen nach Applikation wird das Wachstum der behandelten Pflanzen im Vergleich zu gestützten, aber unbehandelten Kontrollpflanzen verglichen. Dabei ergibt sich folgendes Resultat:

| Behandlung mit Verbindung Nr. | Menge in kg AS/ha | Kultur | Nachwuchs nach Behandlung (Pflanzenhöhe) |
|---|---|---|---|
| Unbehandelt | | | 100 % |
| 1.001 | 0.3 | Centrosema pubescens | 100 % |
| 1.001 | 1.0 | Centrosema pubescens | 90 % |
| 1.001 | 3.0 | Centrosema pubescens | 20 % |
| 1.001 | 0.3 | Psophocarpus palustris | 90 % |
| 1.001 | 1.0 | Psophocarpus palustris | 30 % |
| 1.001 | 3.0 | Psophocarpus palustris | 10 % |

0175652

Patentansprüche

1. 5-Acylaminopyrimidine der allgemeinen Formel I

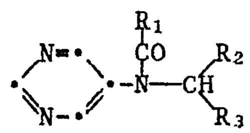

(I)

worin

$R_1$ für $C_{1-8}$-Alkyl, Halogen-$C_{1-6}$-alkyl, $C_{1-6}$-Alkoxy, $C_{1-4}$-Alkoxy-$C_{1-8}$-alkyl, Thio-$C_{1-4}$-alkyl, Halogen; Phenyl oder Phenoxy, gegebenenfalls jeweils substituiert durch Halogen, $C_{1-4}$-Alkyl, Halogen-$C_{1-4}$-alkyl, Carboxy-$C_{1-4}$-alkyl, Halogen-$C_{1-4}$-alkoxy, $C_{1-4}$-Alkoxy, Halogen-$C_{1-4}$-alkylthio, Cyano, Nitro; $C_{2-4}$-Alkenyl, $C_{2-4}$-Alkinyl, $C_{3-6}$-Cycloalkyl, gegebenenfalls substituiert durch Halogen, und $C_{1-4}$-Alkyl, steht,

$R_2$ $C_{1-8}$-Alkyl, gegebenenfalls substituiert durch Halogen, Cyano, $C_{1-4}$-Alkoxy; Phenyl, Phenyl-$C_{1-8}$-alkyl, worin der Phenylkern jeweils gegebenenfalls wie unter $R_1$ definiert, substituiert ist; Pyridyl, Thienyl, Furyl, wobei der Kern wie der Phenylrest unter $R_1$ definiert, gegebenenfalls substituiert ist, Pyranyl, Dihydropyranyl, $C_{3-7}$-Cycloalkyl, $C_{3-7}$-Alkenyl, und

$R_3$ Wasserstoff oder $C_{1-8}$-Alkyl bedeutet und $R_2$ und $R_3$ mit dem sie verbindenden Kohlenstoffatom einen $C_{5-7}$-Cycloalkylrest bilden können.

2. Verbindungen gemäss Anspruch 1, dadurch gekennzeichnet, dass $R_1$ für $C_{1-8}$-Alkyl, $C_{1-6}$-Alkoxy, $C_{1-4}$-Alkoxy-$C_{1-8}$-alkyl, Thioalkyl, Halogen; Phenyl oder Phenoxy, gegebenenfalls jeweils substituiert durch Halogen, Alkyl, Halogenalkyl, Carboxyalkyl, Halogenalkoxy, Halogenalkylthio, Cyano, Nitro; $C_{2-4}$-Alkenyl, $C_{2-4}$-Alkinyl, $C_{3-6}$-Cycloalkyl, gegebenenfalls substituiert durch Halogen, Alkyl, steht,

$R_2$ $C_{1-8}$-Alkyl, gegebenenfalls substituiert durch Halogen, Cyano, Alkoxy; Phenyl, Phenyl-$C_{1-8}$-alkyl, worin der Phenylkern jeweils

gegebenenfalls wie unter $R_1$ definiert, substituiert ist; Pyridyl, Thienyl, Furyl, wobei der Kern wie der Phenylrest unter $R_1$ definiert, gegebenenfalls substituiert ist, und
$R_3$ Wasserstoff oder $C_{1-8}$-Alkyl bedeutet.

3. Verbindungen gemäss Anspruch 1, dadurch gekennzeichnet, dass $R_1$ $C_{2-4}$-Alkyl ist, $R_2$ Phenyl, gegebenenfalls durch Chlor substituiert ist und $R_3$ Wasserstoff ist.

4. Verbindungen gemäss Anspruch 1, dadurch gekennzeichnet, dass $R_1$ Aethyl, Propyl, Isopropyl oder tert-Butyl ist, $R_2$ 4-Chlorphenyl oder 2,4-Dichlorphenyl ist.

5. N-(4-Chlorbenzyl)-N-(pyrimidin-5-yl)-propionsäureamid gemäss Anspruch 1.

6. N-(2,4-Dichlorbenzyl)-N-(pyrimidin-5-yl)-isobuttersäureamid gemäss Anspruch 1.

7. N-(4-Chlorbenzyl)-N-(pyrimidin-5-yl)-trimethylacetamid gemäss Anspruch 1.

8. N-(2,4-Dichlorbenzyl)-N-(pyrimidin-5-yl)-trimethylacetamid gemäss Anspruch 1.

9. N-(4-Brombenzyl)-N-(pyrimidin-5-yl)-trimethylacetamid gemäss Anspruch 1.

10. 5-Aminopyrimidine der allgemeinen Formel II

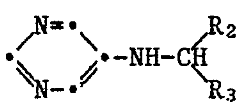

II

11. Verbindungen gemäss Anspruch 10, dadurch gekennzeichnet, dass $R_2$ Phenyl, gegebenenfalls durch Chlor substituiert ist und $R_3$ Wasserstoff ist.

12. Verbindungen gemäss Anspruch 10, dadurch gekennzeichnet, dass $R_2$ 4-Chlorphenyl oder 2,4-Dichlorphenyl ist.

13. Azomethine der allgemeinen Formel III

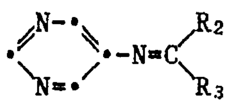 (III)

14. Azomethine gemäss Anspruch 13, dadurch gekennzeichnet, dass $R_2$ Phenyl, gegebenenfalls durch Chlor substituiert ist und $R_3$ Wasserstoff ist.

15. Azomethine gemäss Anspruch 13, dadurch gekennzeichnet, dass $R_2$ 4-Chlorphenyl oder 2,4-Dichlorphenyl ist.

16. Mittel zur Regulierung des Pflanzenwachstums, dadurch gekennzeichnet, dass es neben Träger- und/oder anderen Zuschlagstoffen als Wirkstoff mindestens eine Verbindung der Formel I gemäss Anspruch 1 enthält.

17. Mittel nach Anspruch 16, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensids enthält.

18. Mittel nach Anspruch 17, dadurch gekennzeichnet, dass es 0,1 bis 95 % eines Wirkstoffes der Formel I, 99,8 bis 5 % eines festen oder flüssigen Zusatzstoffes und 0,1 bis 25 % eines Tensides enthält.

19. Verfahren zur Herstellung eines wie in Anspruch 16 beanspruchten 5-Acylaminopyrimidines, dadurch gekennzeichnet, dass man mindestens eine gemäss Anspruch 1 definierte Verbindung der Formel I mit geeigneten festen oder flüssigen Zusatzstoffen und Tensiden innig vermischt.

20. Verfahren zur Regulierung des Pflanzenwachstums, dadurch gekennzeichnet, dass man eine gemäss Anspruch 1 definierte Verbindung der Formel I in einer wirksamen Menge auf die Pflanze oder deren Standort appliziert.

21. Verwendung von Verbindungen der Formel I gemäss Anspruch 1 zur Regulierung des Pflanzenwachstums.

22. Verfahren gemäss Anspruch 20 zur Wuchshemmung im Sinne einer Erhöhung der Knickfestigkeit und Halmverkürzung bei Getreidesorten.

23. Verwendung gemäss Anspruch 22, dadurch gekennzeichnet, dass es sich bei den Getreidesorten um Hafer, Weizen, Gerste oder Roggen handelt.

24. Verfahren gemäss Anspruch 20 zur Wuchshemmung bei Gräsern.

25. Verfahren gemäss Anspruch 20 zur Wuchsregulierung bei Leguminosen im Sinne einer Ertragssteigerung.

26. Verfahren gemäss Anspruch 25, dadurch gekennzeichnet, dass es sich um Soja handelt.

FO 7.5 IS/bg*/jt*